# EUROPEAN PATENT APPLICATION

(11) **EP 1 197 551 A1**
(43) Date of publication of application: **17.04.2002**
(21) Application number: 00944440.7
(22) Date of filing: 17.07.2000
(51) Int. Cl.: C12N 15/00, C12Q 1/68, C12M 1/00, G01N 33/566, G01N 33/53

(54) **DNA CHIP**

(30) Priority: 23.07.1999 JP 20978299
(71) Applicant: Takara Shuzo Co, Ltd., Kyoto-shi, Kyoto 612-8061 (JP)
(72) Inventor: Asada, Kiyozo, Koka-gun, Shiga 520-3333 (JP); Ueda, Minoru, Kusatsu-shi, Shiga 525-0025 (JP); Takayama, Masanori, Kyotanabe-shi, Kyoto 610-0311 (JP); Mineno, Junichi, Uji-shi, Kyoto 611-0002 (JP); Kimizuka, Fusao, Omihachiman--shi, Shiga 523-0056 (JP); Kato, Ikunoshin, Uji-shi, Kyoto 611-0028 (JP)
(74) Representative: Grund, Martin, Dr.
(86) International application number: JP0004791
(87) International publication number: WO0107593

(57) **Abstract**

A DNA chip having DNA arrayed and immobilized in a preliminarily determined domain on the surface of a support, characterized in that double-stranded DNA is immobilized onto the immobilization support under denaturation conditions.

## Description

### Technical Field

The present invention relates to a highly sensitive DNA chip, which is useful in a field of gene analysis. The present invention also relates to a DNA solvent for the preparation of the chip and a method for preparing the chip.

### Background Art

With the progress of projects for analyzing genomes, the entire nucleotide sequences of genomes of various organisms have been elucidated. New projects are proceeding for examining gene expression patterns and functions of gene products on a level of whole genome based on the information on the nucleotide sequences.

DNA chips were developed as means for remarkably promoting such function analysis projects. The DNA chip techniques have already been used for analyzing the expression of all of the yeast genes, or genes in cultured cells or organs.

Known methods for preparing DNA chips (or DNA arrays) include, for example, a method in which a single-stranded DNA is synthesized in a predetermined region on a chip solid phase, and a method in which a single-stranded or double-stranded DNA which has been prepared beforehand is spotted within a predetermined region on a chip solid phase. DNA chips prepared according to the former method are exemplified by high-density oligonucleotide arrays as described in United States Patent No. 5,445,934, 5,744,305 and 5,700,637. DNA chips prepared according to the latter method are exemplified by spotting arrays as described in United States Patent No. 5,807,522 and WO 98/18961. However, a large-scale instrument for solid phase synthesis at high density is required for the preparation of the above-mentioned high-density oligonucleotide array. Furthermore, it is difficult to synthesize a long nucleotide chain according to this method because of its synthesis yield. In case of a spotting array having double-stranded DNAs being spotted, a step of denaturing the double-stranded DNAs immobilized on the DNA chip into single-stranded DNAs before use is indispensable; making the analysis procedure complicated. In case of a spotting array having single-stranded DNAs being spotted, it is required to prepare the single-stranded DNAs from double-stranded DNAs before immobilizing them onto a solid phase, making the chip preparation procedure complicated. As described above, conventional DNA chips and conventional methods for preparing such DNA chips had several problems.

In addition, it is desired to provide DNA chips onto which DNAs containing open reading frames (hereinafter referred to as ORFs) on a level of whole genome or fragments thereof are arrayed and immobilized in order to determine gene expression patterns or functions of gene products on a level of whole genome of an organism. However, DNA chips having DNAs of varying chain length being arrayed and immobilized have problems such as low immobilization rate for short DNAs and weak hybridization signals. Thus, it is desired to provide DNA chips that result in high immobilization rate and strong hybridization signals.

### Objects of Invention

The main object of the present invention is to provide a highly sensitive DNA chip which can be used to monitor the expression of genes in an organism on a level of whole genome.

### Summary of Invention

The present invention is outlined as follows. The first aspect of the present invention relates to a DNA chip having DNAs being arrayed and immobilized in predetermined regions on a surface of a substrate, which is prepared by a method comprising immobilizing double-stranded DNAs onto an immobilization substrate under denaturing conditions.

In the first aspect, a DNA solvent containing a volatile acid or a salt thereof can be preferably used for immobilizing the DNAs onto the immobilization substrate. Although it is not intended to limit the present invention, a DNA solvent containing a volatile organic acid or a salt thereof is preferably used. More preferably, the DNA solvent contains acetic acid or a salt thereof. The present invention provides a DNA chip having DNAs being arrayed and immobilized in predetermined regions, wherein the DNAs immobilized on an immobilization substrate are at least partially single-stranded. The immobilization substrate used in the first aspect is not specifically limited as long as it can be used to immobilize DNAs at high density. A nonporous substrate is preferably used. Although it is not intended to limit the present invention, for example, the nonporous substrate is preferably made of glass.

The second aspect of the present invention relates to a DNA solvent for preparing a DNA chip containing a volatile acid or a salt thereof. In the second aspect, the volatile acid or a salt thereof is preferably a volatile organic acid or a salt thereof. Although it is not intended to limit the present invention, the volatile organic acid or a salt thereof is preferably acetic acid or a salt thereof.

The third aspect of the present invention relates to a method for preparing a single-stranded DNA chip, the method comprising arraying and immobilizing DNAs in predetermined regions on an immobilization substrate using a DNA solvent containing a volatile acid or a salt thereof. In the third aspect, the volatile acid or a salt thereof is preferably a volatile organic acid or a salt thereof. Although it is not intended to limit the present invention, the volatile organic acid or a salt thereof is preferably acetic acid or a salt thereof. Furthermore, the immobilization substrate used in the third aspect is preferably a nonporous substrate. For example, the nonporous substrate is made of glass.

The present inventors studied intensively to develop a DNA chip which can be used to monitor the expression of genes in an organism on a level of whole genome, and found a method that enables efficient immobilization of DNAs. Furthermore, the present inventors successfully developed a highly sensitive DNA chip which results in high hybridization efficiency and low background using the method. Thus, the present invention has been completed.

### Detailed Description of the Invention

As used herein, a DNA chip refers to one having double-stranded DNAs being arrayed and immobilized in predetermined regions on a surface of a substrate under denaturing conditions, wherein the immobilized DNAs are at least partially single-stranded. The DNA chip is also called as a DNA microarray. As used herein, a group of DNAs being arrayed and immobilized in predetermined regions on an immobilization substrate may be referred to as an array. As used herein, a high-density DNA chip refers to a DNA chip having DNAs being arrayed and immobilized in predetermined regions on a surface of a substrate at density of 100 spots/cm² or more.

According to the present invention, any volatile acid may be preferably used as long as the acid component can be volatilized when water is evaporated during a series steps (e.g., during a step of drying DNAs spotted onto a substrate) in a course of arraying and immobilizing the DNAs in predetermined regions on a surface of the substrate. Any volatile acids including inorganic acids and organic acids may be preferably used without limitation. For example, acetic acid is preferable.

According to the present invention, a salt of a volatile acid is a salt of the acid as described above. For example, salts with bases classified into the group 1 or 2 in the periodic table of elements are preferable.

As used herein, an ORF refers to a DNA sequence that encodes or may possibly encode a protein among genes being coded for in a genome of an organism.

### (1) The DNA chip of the present invention

A DNA to be arrayed and immobilized in a predetermined region on a surface of a substrate of the DNA chip of the present invention is not specifically limited. A double-stranded polynucleotide of 50 bases or more in length or a derivative thereof is generally used. For example, a preferable DNA is prepared by conducting enzymatic amplification using a polymerase chain reaction or the like and denaturing the amplification product upon immobilization onto a substrate to convert it into single-stranded DNAs or derivatives thereof. For example, one having a modification that enables immobilization onto a surface of a substrate can be used as a derivative. Although it is not intended to limit the present invention, derivatives are exemplified by a DNA having a functional group such as an amino group or a thiol group being introduced at the 5'-terminus of the DNA.

A DNA to be immobilized in a predetermined region on a substrate of the DNA chip of the present invention may be consisting of a sense strand and an antisense strand from one DNA fragment, or those from two or more DNA fragments.

Although it is not intended to limit the present invention, a DNA chip in which a mixture of double-stranded DNAs having nucleotide sequences derived from plural different portions in one gene are immobilized in a predetermined region is exemplified.

According to the present invention, a substrate is not specifically limited and may be made of a nonporous material having a smooth surface. For example, one made from glass such as a slide glass is preferably used. There is no specific limitation regarding the surface of the substrate as long as a single-stranded DNA can be immobilized on it through a covalent or noncovalent bond. For example, a substrate having a hydrophilic or hydrophobic functional group on its surface is preferably used. Examples of substrates that can be preferably used include, but are not limited to, those having a hydroxyl group, an amino group, a thiol group, an aldehyde group, a carboxyl group, an acyl group or the like. Such a functional group may be present as a property of the surface of the substrate itself. Alternatively, it may be introduced by surface treatment. Examples of substrates subjected to such surface treatment include glass treated with a commercially available silane-coupling agent such as aminoalkylsilane, or with a polycation such as polylysine or polyethylenimine. Slide glasses having been subjected to some of the above-mentioned treatments are commercially available.

As used herein, denaturation upon immobilization means that a DNA to be immobilized which is dissolved in an appropriate solvent is maintained under conditions in which a double-stranded DNA is dissociated into single-stranded DNAs. For example, treatments such as heat-denaturation and alkaline-denaturation are included. The DNA that has been immobilized onto an immobilization substrate under such denaturing conditions is at least partially single-stranded.

In another embodiment, a DNA chip prepared using a DNA solvent for preparing a DNA chip containing a volatile acid or a salt thereof is exemplified. When such a DNA chip is prepared, the acid component is volatilized upon drying after spotting, and the pH is consequently elevated. As a result, a DNA chip is prepared. In the DNA chip, double-stranded DNAs contained in such a solvent are arrayed and immobilized, and are at least partially single-stranded.

Furthermore, the DNA chip of the present invention may be used after boiling in boiling water and then rapidly cooling in cold ethanol on ice bath.

### (2) The DNA solvent for preparing a DNA chip of the present invention

As the DNA solvent for preparing a DNA chip of the present invention, one containing a volatile acid or a salt thereof is preferably used. Both of organic acids and inorganic acids may be preferably used as volatile acids. Organic acids are not specifically limited as long as they are volatile, and exemplified by carboxylic acids such as formic acid and acetic acid. Inorganic acids are not specifically limited as long as they are volatile, and exemplified by carbonic acid, cyanic acid and hydrocyanic acid. Any volatile acid may be preferably used as long as the acid component can be volatilized when water is evaporated during a series steps (e.g., during a step of drying DNAs spotted onto a substrate) in a course of arraying and immobilizing the DNAs in predetermined regions on a surface of the substrate.

According to the present invention, the above-mentioned volatile acid may be a salt. Although it is not intended to limit the present invention, salts with ones classified into the group 1 (lithium, sodium, potassium, etc.) or the group 2 (beryllium, magnesium, calcium, etc.) in the periodic table of elements are exemplified.

The DNA solvent for preparing a DNA chip of the present invention is not specifically limited and exemplified by a DNA solvent containing sodium acetate. If a sodium acetate solution is used in the present invention, the concentration of sodium acetate is 50 mM or more, preferably 200 mM or more, more preferably 300 mM or more. The pH of sodium acetate solution is preferably from 5.0 to 7.0, more preferably from 5.5 to 6.5. If a DNA solvent containing sodium acetate prepared as described above is used in the method for preparing a DNA chip of the present invention, acetic acid is volatilized upon drying after spotting, and the pH is consequently elevated. As a result, a DNA chip is prepared. In the DNA chip, double-stranded DNAs contained in such a solvent are arrayed and immobilized in predetermined regions on a surface of a substrate, and are at least partially single-stranded.

Furthermore, the DNA solvent of the present invention may contain a surfactant. Although it is not intended to limit the present invention, for example, a non-ionic surfactant such as sucrose monolaurate is preferably used. The concentration of sucrose monolaurate is, without limitation, preferably from 0.005% to 0.05%, more preferably from 0.01% to 0.03%.

The DNA solvent for preparing a DNA chip of the present invention may contain a DNA consisting of a sense strand and an antisense strand from one DNA fragment, or those from two or more DNA fragments. Although it is not intended to limit the present invention, a DNA solvent for preparing a DNA chip containing a mixture of double-stranded DNAs having nucleotide sequences derived from plural different portions in one gene is exemplified.

The chain length of the DNA to be contained in the DNA solvent for preparing a DNA chip of the present invention is not specifically limited. A DNA of 50 bases or more in length is preferable.

### (3) The method for preparing DNA chip of the present invention

The chain length of the DNA fragment to be immobilized according to the method for preparing a DNA chip of the present invention is not specifically limited. A DNA of 50 bases or more in length is preferable. According to the preparation method of the present invention, the DNA fragment to be immobilized may be a DNA fragment obtained by excising from a plasmid having the DNA fragment of interest being inserted or the like using a restriction enzyme. Alternatively, it may be a DNA fragment prepared using a nucleic acid amplification method such as a PCR.

According to the method for preparing a DNA chip of the present invention, the DNA to be immobilized in a predetermined region on a substrate may be consisting of sense and antisense strands from one DNA fragment, or those from two or more DNA fragments. Although it is not intended to limit the present invention, a mixture of double-stranded DNAs having nucleotide sequences derived from plural different portions in one gene is immobilized in a predetermined region in a exemplary preparation method. In another exemplary preparation method, sense and antisense strands from plural DNA fragments are spotted one by one in the same predetermined region.

The method for preparing a DNA chip, of the present invention is specifically illustrated with reference to DNAs from a genome of an organism.

For example, an ORF contained in a genome of an organism or a fragment thereof is prepared as follows. An amplification reaction by a PCR is carried out based on information on a nucleotide sequence of an ORF contained in a database published through the Internet or originally determined. A genomic DNA, a cDNA library or the like is used as a template in the amplification reaction. It is known that the efficiency of a PCR is generally reduced if the length of the fragment to be amplified exceeds 1000 base pairs (bp). On the other hand, it is known that the DNA immobilization rate upon UV crosslinking land the detection sensitivity of a DNA chip are reduced if the DNA fragment to be immobilized is shorter than 300 bp. Thus, primers are generally designed such that the length of a DNA region to be amplified for the preparation of a DNA chip ranges from 300 to 1000 bp. However, the present invention increases the immobilization rate for DNAs of less than 300 bp and the detection sensitivity of a DNA, and enables the preparation and use of a DNA chip comprising DNAs of less than 300 bp on a level of whole genome. Thus, a primer pair that amplifies a DNA region of less than 300 bp in length can be designed for the preparation of a DNA chip according to the present invention.

A primer is designed such that it appropriately anneals to a template. Although it is not intended to limit the present invention, the primer is preferably 20 bases or more in length, for example. Furthermore, it is preferable to design the primer to have GC content of about 50 to 60%. For example, a primer of less than 20 bases in length or having GC content outside the above-mentioned range can be used without problem as long as it can be used for amplification by a PCR.

A primer designed as described above can be synthesized using a DNA synthesizer or the like. A template DNA can be prepared according to a known method for preparing a genomic DNA or a cDNA library. A reaction mixture having a composition and a reaction cycle commonly used are used for amplifying an ORF or a fragment thereof by a PCR. If a number of reactions are simultaneously carried out in parallel, it is preferable to conduct a PCR in a 96-well plate. In fact, an instrument used for such a reaction is commercially available.

If a reaction mixture containing bovine serum albumin (BSA) is used for amplification by a PCR, it is preferable to make the BSA concentration lower than that conventionally used. The final concentration of BSA is, without limitation, preferably less than 0.01%(w/v), more preferably 0.005% or less, more preferably 0.001% or less.

A conventional method for purifying a DNA fragment is used for the purification of a DNA fragment amplified by a PCR (a PCR product). A commercially available purification kit in a 96-well format such as QIAquick PCR Purification Kit (Qiagen) may be used in order to simultaneously purify a number of samples in parallel. Since the salt concentration of the purified DNA solution influences homogeneity of a spot, it is necessary to purify the DNA fragment such that it is sufficiently desalted. The DNA fragment may be purified according to an isopropanol precipitation method. A DNA fragment obtained by conducting a PCR using a low BSA concentration as described above can be used after a brief purification procedure. Next, the size, the purity and the yield of the amplification product are determined using a portion of the purified DNA fragment. The size of the amplification product is determined by electrophoresis on 2 or 3% agarose gel. The purity is determined by analyzing the electrophoretic pattern of the gel, for example, using an image analyzer or an electrophoresis gel analysis system to calculate the relative intensity of the main band. Additionally, the DNA concentration is determined according to a UV absorbance method or a fluorophotometric method.

The DNA fragment lyophilized in a 96-well plate is dissolved in a given solvent for immobilization at a given concentration with reference to the measurement results. For example, 3 x SSC (a sodium chloride/sodium citrate solution; 1 x SSC is 0.15 M sodium chloride/0.015 M sodium citrate) is used as a solvent for immobilization. Although it is not intended to limit the present invention, for example, a DNA solvent containing a volatile acid or a salt thereof is preferably used according to the present invention. An organic acid such as acetic acid or an inorganic acid such as carbonic acid may be preferably used as a volatile acid. A surfactant (e.g., a non-ionic surfactant) may be added to the DNA solvent. An example of the surfactant is sucrose monolaurate.

In another embodiment, a buffer containing morpholine, a morpholine derivative, a salt thereof or a carbonate may be used (Japanese Patent Application No. 10-351276). In this case, the salt concentration of the solvent for immobilization is preferably from 10 to 500 mM, more preferably from 50 to 200 mM. Increase in DNA immobilization rate contributes to increase in detection sensitivity of a DNA chip. Therefore, it is preferable to use such a buffer which results in high immobilization efficiency particularly for a chip to be used for monitoring the expression of genes on a level of whole genome. On the other hand, the amount of immobilized DNA is generally increased as the concentration of the DNA to be immobilized onto a substrate is increased. However, the amount of available DNA is limited. Thus, it is preferable to adjust the concentration to be as constant as possible and 0.1 mg/ml or more. Thereby, the amount of the immobilized DNA becomes constant, and a suitable chip for gene expression analysis can be prepared.

A DNA fragment dissolved in a solvent for immobilization as described above is subjected to heat- or alkali-denaturation, and then spotted onto a substrate using a commercially available arrayer (an instrument for preparing DNA chips). This denaturation is very useful as means of increasing the detection sensitivity of a chip because it increases the DNA immobilization rate. The immobilization rate for a short DNA of less than 300 bp is particularly increased. Therefore, the method of the present invention is effective if a DNA chip on a level of whole genome of an organism is prepared. This is because such a DNA chip contains ORFs and, for many of such ORFs, DNA fragments to be immobilized are of about 100 bp in length. Heat denaturation is carried out, for example, by maintaining a DNA solution in boiling water for 1 to 2 minutes and then rapidly cooling it. Alternatively, denaturation may be alkali-denaturation in which pH of a DNA solution is elevated and then the solution is neutralized. For example, sodium hydroxide at a final concentration of 0.2 M is added to a DNA solution, and the mixture is incubated at room temperature for 5 minutes and then neutralized by adding sodium acetate (pH 5) at a final concentration of 0.3 M. The DNA chip of the present invention is used to efficiently immobilized single-stranded DNAs under denaturing conditions as described above and results in strong hybridization signals.

Furthermore, according to the present invention, a solution of a salt of a volatile acid such as a salt of a volatile organic acid with a strong base (e.g., sodium acetate buffer (pH 6.0)) is used as a solvent. When a double-stranded DNA dissolved in the solution is placed on a substrate, the pH of the solution is elevated as a result of evaporation of the solvent, resulting in the denaturation of the DNA. Thus, a DNA chip can be prepared, wherein the DNAs are at least partially single-stranded. Such a DNA chip can be used as effectively as one prepared by placing single-stranded DNAs. A salt of a volatile organic acid with a strong base may be added to the above-mentioned solution for immobilization.

A commercially available arrayer can be used in the preparation method of the present invention as long as it can be used to prepare a homogenous high-density DNA chip with reproducibility in a short time. For example, 417 Arrayer from Genetic Microsystems (GMS) can be used. The size of spotting is usually less than 1 nanoliter. The space of spotting is usually about 300 µm. Using an arrayer having such specification, all ORFs in a genome are readily arrayed on one slide glass if the genome contains 5000 or less ORFs.

A substrate onto which DNAs have been spotted using an arrayer is incubated, for example, at a temperature from 25 to 50°C for 1 hour or more in humidity from 80 to 100% in a humidified incubator. Then, immobilization can be suitably completed by ultraviolet irradiation. Naturally, the humidity, the temperature and the period in the humidified incubator are optimized depending on the atmosphere temperature because they are closely related to the atmosphere temperature. The substrate having the DNAs being immobilized is washed in an aqueous solution containing a suitable surfactant such as 0.2% sodium dodecyl sulfate (SDS) followed by distilled water. Functional groups on the surface of the substrate which are not participated in the immobilization are then blocked.

The quality of a DNA chip prepared as described above can be assessed, for example, by hybridization with a fluorescence-labeled genomic DNA fragment.

According to the present invention, a DNA chip having DNAs being immobilized in which the DNAs are at least partially single-stranded can be prepared. This is accomplished by spotting a double-stranded DNA to be immobilized onto a substrate after denaturing it into single-stranded DNAs. As a result, the immobilization rate of the DNA is increased. Thus, the chip has a high detection sensitivity. Furthermore, the method of the present invention is particularly useful as a method for immobilization with high immobilization rate if short DNAs of less than 300 bp, in particular of about 100 bp, are to be immobilized onto a substrate.

Since a double-stranded DNA is immobilized onto a immobilization substrate under denaturing conditions according to the present invention, the present invention is also useful for monitoring the expression of both of the sense and antisense strands of a gene.

### Examples

The following examples further illustrate the present invention in detail but are not to be construed to limit the scope thereof.

### Example 1: Preparation and detection sensitivity of DNA chip having denatured DNAs being arrayed and immobilized

DNA chips were prepared by spotting native or denatured DNAs. Then, the intensities of hybridization signals were compared.

A cDNA fragment for human cancer-associated gene CA13 as a test sample DNA was prepared as follows. Briefly, a PCR was carried out using a cDNA from human stomach tissue as a template and primers CA13-S6 and CA13-A4 having nucleotide sequences of SEQ ID NOS:1 and 2 as primers according to the method as described in WO 98/37187. A CA13 cDNA fragment of about 1.0 kb having a nucleotide sequence of SEQ ID NO:10 was prepared. The CA13 fragment was purified by removal of the primers using Microcon-100 (Amicon) followed by ethanol precipitation. The concentration of a portion of the purified DNA dissolved in 1 x SSC, 3 x SSC or 50 mM carbonate buffer (pH 9.5) was determined by measuring the absorbance at 260 nm. Then, solutions each containing the purified DNA in one of the above-mentioned solvents at a concentration of 0.1 or 0.5 mg/ml were prepared. Samples to be subjected to alkali denaturation as described below were prepared such that the treatment would result in the above-mentioned concentrations.

The respective samples were incubated at room temperature for 5 minutes after adding NaOH at a final concentration of 0.2 M, and neutralized by adding sodium acetate (pH 5.0) at a final concentration of 0.3 M. Hereinafter, this procedure is referred to as alkaline denaturation. The DNAs with or without denaturation were spotted onto a slide glass using GMS 417 Arrayer (GMS). POLY-PREP-SLIDES (Sigma, hereinafter referred to as SIG/PLL) and MAS-coated slide glass (Matsunami Glass; hereinafter referred to as MAT/MAS) were used as immobilization substrates. The size of spotting was 150 µm. The space of spotting was 375 µm. Three spots were arrayed for one DNA.

After spotting, each of the slide glass was incubated at 37°C in humidity of 90% for 1 hour in a humidified incubator KCL1000 (EYELA), and then subjected to UV crosslinking at an energy of 60 mJ/cm² using a ultraviolet irradiation apparatus FS-1500 (Funakoshi). Each of the slide glass was washed once in 0.2% SDS followed by twice in distilled water, centrifuged at a low speed of about 1,000 rpm to remove water, and air-dried. The amino groups on the surface were then inactivated by succinylation as follows. First, a blocking solution was prepared by dissolving 1.5 g of succinic anhydride in 89.5 ml of N-methyl-2-pyrrolidone, and mixing the solution with 9 ml of 1 M borate buffer (pH 8.0). The slide glass was immediately soaked in the blocking solution and treated at room temperature for 20 minutes. After treatment, the slide glass was washed extensively in distilled water, centrifuged at a low speed to remove water, and air-dried.

Next, a 1.0-kb CA13 fragment labeled with Cy5 (Amersham Pharmacia) was prepared. Briefly, a PCR was carried out in a similar manner as described above for the preparation of the CA13 fragment except that a Cy5-labeled primer CA13-A4 was used in place of the primer CA13-A4. The thus obtained Cy5-labeled CA13 fragment was purified as described above, and then dissolved in TE buffer (10 mM tris-hydrochloride (pH 8.0, Nacalai Tesque) containing 1 mM EDTA (Nacalai Tesque)).

After blocking, some of the slide glasses were boiled in boiling water for 3 minutes, rapidly cooled in ethanol bath on ice bath, and then dried by low-speed centrifugation. Hereinafter, this procedure is referred to as heat denaturation. Hybridization was carried out as follows. A hybridization solution containing 1 µM Cy5-labeled CA13 fragment, 4 x SSC, 0.2% SDS and 100 µg/ml salmon sperm DNA was dripped onto the array on each slide glass. The drop was covered with a cover glass such that no air bubble was included. The sides of the cover glass were sealed with a sealing film and the slide glass was incubated at 60°C overnight. After hybridization, the cover glass was removed from the slide glass in 2 x SSC. The slide glass was washed in 2 x SSC containing 0.2% SDS for 5 minutes followed by 0.05 x SSC for 5 minutes at room temperature. The slide glass was then dried by low-speed centrifugation. The slide glass was subjected to a measurement using GMS 418 Array Scanner (GMS) at an excitation wavelength of 635 nm and an emission wavelength of 660 nm. The relative intensity of hybridization signal was analyzed using an image analysis software ImaGene (BioDiscovery). The results are shown in Table 1. Table 1 shows the effects of the alkali denaturation of spotted DNAs upon preparation of DNA chips. The relative intensity of signal indicated in Table 1 represents an average of relative intensities of signals from three spots.

**Table 1**

| Substrate | Heat | DNA concentration | Solvent | Relative intensity of signal | |
|---|---|---|---|---|---|
| denaturation (mg/ml) | | | | Alkali denaturation | |
| | | | | + | - |
| MAT/MAS | + | 0.5 | 1 x SSC | 9261 | 755 |
| | | | 3 x SSC | 7524 | 3083 |
| | | | Carbonate | 9482 | 5526 |
| | | 0.1 | 1 x SSC | 2934 | 49 |
| | | | 3 x SSC | 2547 | 327 |
| | | | Carbonate | 2676 | 1114 |
| MAT/MAS | - | 0.5 | 1 x SSC | 8328 | 6042 |
| | | | 3 x SSC | 6674 | 4109 |
| | | | Carbonate | 9183 | 6229 |
| | | 0.1 | 1 x SSC | 3176 | 2671 |
| | | | 3 x SSC | 2975 | 1148 |
| | | | Carbonate | 2195 | 1848 |
| SIG /PLL | + | 0.5 | 1 x SSC | 2196 | 1149 |
| | | | 3 x SSC | 1583 | 787 |
| | | | Carbonate | 2504 | 1173 |
| | | 0.1 | 1 x SSC | 463 | 414 |
| | | | 3 x SSC | 372 | 248 |
| | | | Carbonate | 443 | 280 |
| SIG/PLL | - | 0.5 | 1 x SSC | 4076 | 1159 |
| | | | 3 x SSC | 3558 | 993 |
| | | | Carbonate | 5372 | 2125 |
| | | 0.1 | 1 x SSC | 834 | 641 |
| | | | 3 x SSC | 831 | 235 |
| | | | Carbonate | 978 | 491 |

As shown in Table 1, for each slide glass, a high relative intensity of hybridization signal was observed when a sample was spotted under alkali-denaturing conditions regardless of the heat denaturation after immobilization. In particular, it was confirmed that a stronger hybridization signal can be achieved by conducting alkali denaturation even if a carbonate buffer was used. The carbonate buffer had been conventionally recognized to result in high immobilization efficiency. Thus, when a double-stranded DNA is spotted after denaturing to make it at least partially single-stranded, the denatured DNA can be efficiently immobilized onto a substrate, resulting in a strong hybridization signal. The results for the heat-denatured slide glass shows that the effects observed when an alkali-denatured DNA is spotted are not observed even if a double-stranded DNA is denatured after spotting. Therefore, it is particularly important to spot a double-stranded DNA under alkali-denaturing conditions.

### Example 2: Influence of DNA size on chip having denatured DNA being immobilized

DNA chips having native or denatured double-stranded DNAs of varying length being spotted were prepared, and the intensities of hybridization signals were compared. A cDNA fragment for human transferrin receptor (hereinafter referred to as TFR) as a test sample DNA was prepared as follows. First, mRNA extraction from human K562 cells (Dainippon Pharmaceutical) and cDNA synthesis were carried out according to conventional methods. A PCR was carried out using the resulting cDNA as a template and primers S7 (SEQ ID NO:3) and AS7 (SEQ ID NO:4) specific for TFR (GenBank No. X01060) as primers. A PCR product encoding the entire 4,738-bp region was obtained. The product was ligated to a vector pT7Blue (Novagen) to obtain a recombinant plasmid pT7TFR. PCRs were carried out using pT7TFR as a template and one of the following primer pairs as primers: S7 and AS6 (SEQ ID NO:5); S7 and AS4 (SEQ ID NO:6); S7 and AS3 (SEQ ID NO:7); S7 and AS2 (SEQ ID NO:8); and S7 and AS1 (SEQ ID NO:9). Thus, TFR fragments of 2.0, 1.0, 0.5, 0.2 and 0.1 kb were prepared and purified by ethanol precipitation. The purified DNAs were dissolved in 50 mM carbonate buffer (pH 9.5). The concentrations were measured using PicoGreen Kit (Molecular Probes), which can be used to specifically quantify double-stranded DNAs, and adjusted to 0.25, 0.5 or 1.0 mg/ml. Samples to be subjected to alkali denaturation were prepared such that the treatment would result in the above-mentioned concentrations.

The respective samples were spotted onto slide glasses using GMS 417 Arrayer (GMS) as they were or under alkali-denaturing conditions. A PLL-coated slide glass (Matsunami Glass; hereinafter referred to as MAT/PLL) or MAT/MAS was used as an immobilization substrate. The size of spotting was 150 µm. The space of spotting was 375 µm. Two spots were arrayed for one DNA.

A TFR fragment prepared using the primer AS7 labeled with Rhodamine X (hereinafter referred to as ROX) at the 5'-terminus was used as a probe. Hybridization was carried out as follows. A hybridization solution containing the probe at 1 µM, 4 x SSC, 0.2% SDS and 100 µg/ml salmon sperm DNA was dripped onto the array on each slide glass. The drop was covered with a cover glass such that no air bubble was included. The sides of the cover glass were sealed with a sealing film and each of the slide glass was incubated at 42°C for 5 hours. After hybridization, the cover glass was removed from the slide glass in 2 x SSC. The slide glass was washed in 2 x SSC containing 0.2% SDS for 5 minutes followed by 0.2 x SSC for 5.minutes at room temperature. Each of the slide glass was. then dried by low-speed centrifugation. Each of the slide glass was subjected to a measurement using GMS 418 Array Scanner (GMS) at an excitation wavelength of 532 nm and an emission wavelength of 570 nm. The relative intensity of hybridization signal was analyzed using ImaGene. The results are shown in Table 2.

**Table 2**

| Substrate | DNA concentration (mg/ml) | DNA size (bp) | Relative intensity of signal | |
|---|---|---|---|---|
| | | | Alkali denaturation | |
| | | | + | - |
| MAT/MAS | 0.25 | 2000 | 4356 | 2652 |
| | | 1000 | 2597 | 2126 |
| | | 500 | 2295 | 1937 |
| | | 200 | 2939 | 2291 |
| | | 100 | 3095 | 2042 |
| | | | | |
| | 0.50 | 2000 | 6927 | 4305 |
| | | 1000 | 4619 | 3559 |
| | | 500 | 3400 | 3281 |
| | | 200 | 6056 | 3240 |
| | | 100 | 8821 | 3857 |
| | | | | |
| | 1.00 | 2000 | 8384 | 6535 |
| | | 1000 | 6427 | 4546 |
| | | 500 | 5517 | 3849 |
| | | 200 | 11252 | 4833 |
| | | 100 | 12932 | 5053 |
| | | | | |
| MAT/PLL | 0.25 | 2000 | 6470 | 938 |
| | | 1000 | 2280 | 1233 |
| | | 500 | 2042 | 962 |
| | | 200 | 1495 | 682 |
| | | 100 | 1343 | 325 |
| | | | | |
| | 0.50 | 2000 | 10983 | 3257 |
| | | 1000 | 4419 | 980 |
| | | 500 | 2801 | 831 |
| | | 200 | 2624 | 742 |
| | | 100 | 2313 | 1473 |
| | | | | |
| | 1.00 | 2000 | 12165 | 5386 |
| | | 1000 | 7357 | 2397 |
| | | 500 | 2944 | 1087 |
| | | 200 | 3678 | 680 |
| | | 100 | 3686 | 952 |

As shown in Table 2, for each slide glass, a high relative intensity of hybridization signal was observed for a sample in which a double-stranded DNA was spotted onto an immobilization substrate under denaturing conditions regardless of the size of the spotted DNA. The relative intensity of spotting signal was remarkably increased particularly when a short DNA was used at a high concentration. Thus, when a double-stranded DNA is spotted onto an immobilization substrate under denaturing conditions to make the immobilized DNA at least partially single-stranded, a short DNA can be efficiently immobilized onto a substrate, resulting in a strong hybridization signal. The MAT/MAS slide tends to result in a stronger signal than a signal observed using a PLL-coated slide glass such as MAT/PLL. It was confirmed that this slide glass is effective particularly when a DNA chip having short DNAs being spotted is prepared.

### Example 3: Examination on DNA solvent

### (1) Effect of denaturing DNA of sodium acetate solution

A DNA chip was prepared by spotting native double-stranded DNAs dissolved in solvents containing sodium acetate buffer (pH 6.0) at varying concentrations. Amounts of hybridizable DNAs were then compared. A: 1.5-kb TFR fragment as a test sample DNA was prepared as follows. A PCR was carried out using pT7TFR prepared in Example 2 as a template and primers S7 and AS5 having the nucleotide sequence of SEQ ID NO:11 as primers. Thus, a 1.5-kb TFR fragment was prepared and purified by ethanol precipitation. The purified DNA was dissolved in water, and the concentration was determined by measuring the absorbance at 260 nm. Sodium acetate buffer (pH 6.0) was added at a final concentration of 50, 100, 200, 300 or 500 mM to a solution containing the DNA (final concentration of 0.3 mg/ml). 3 x SSC containing the DNA at the same concentration with or without alkali denaturation were used as controls.

The respective samples were spotted onto a MAT/MAS slide glass using GMS 417 Arrayer. After arraying, the slide glass was subjected to immobilization and blocking of the amino groups on its surface as described in Example 1. The slide glass was not subjected to heat denaturation.

Salmon sperm DNA which had been converted into smaller molecules (200 to 2,500 bp, Funakoshi) was labeled with Cy3 using Label IT Cy3 Labeling Kit (Takara Shuzo) according to the instructions attached to the kit. The Cy3-labeled salmon sperm DNA as a probe was hybridized to the DNAs on the slide glass under moderate conditions. The fluorescence intensity of the nonspecifically hybridized probe was measured. Thereby, the amount of hybridizable spotted DNA, i.e., the amount of DNA existing as a single-stranded one was measured. Specifically, hybridization and washing were carried out as described in Example 1 for an oligo DNA probe using 100 ng of the Cy3-labeled salmon sperm DNA as a probe. The slide glass was subjected to a measurement using GMS 418 Array Scanner at an excitation wavelength of 532 nm and an emission wavelength of 570 nm. The relative intensity of hybridization signal was analyzed using ImaGene. The results are shown in Table 3.

**Table 3**

| Solvent | Concentration (mM) | Relative intensity of signal |
|---|---|---|
| Sodium acetate | 50 | 16147 |
| | 100 | 11640 |
| | 200 | 19070 |
| | 300 | 38700 |
| | 500 | 56636 |
| 3 x SSC | | 13972 |
| Alkali denaturation | | 53370 |

As shown in Table 3, the relative intensity of signal was increased as the sodium acetate concentration was increased, confirming that the ratio of hybridizable DNA among the spotted DNAs was increased.

### (2) Effect of addition of surfactant

The effect of further adding a surfactant to the sodium acetate solution was examined. Sucrose monolaurate (Dojindo Laboratories) was used as a surfactant. The examination was carried out by adding sucrose monolaurate at a concentration ranging from 0.005% to 0.05%. A DNA chip was prepared and signals were analyzed as described in (1) above. As a result, it was confirmed that the addition of sucrose monolaurate made the signal intensity in the spot homogeneous and improved the signal intensity. In particular, remarkable improvement was observed at a concentration ranging from 0.01% to 0.03%.

### Industrial Applicability

The present invention provides a DNA chip which results in high immobilization rate for short DNAs and high sensitivity, and which can be used to monitor the expression of genes in an organism on a level of whole genome with high sensitivity and high efficiency. The present invention also provides a DNA solvent for preparing said chip and a method for preparing a DNA chip using said solvent.

### Sequence Listing Free Text

SEQ ID NO:1: Designed oligonucleotide primer designated as CA13-S6 for amplifying cDNA for human cancer-associated gene CA13.

SEQ ID NO:2: Designed oligonucleotide primer designated as CA13-A4 for amplifying cDNA for human cancer-associated gene CA13.

SEQ ID NO:3: Designed oligonucleotide primer designated as S7 for amplifying human transferrin receptor cDNA.

SEQ ID NO:4: Designed oligonucleotide primer designated as AS7 for amplifying human transferrin receptor cDNA.

SEQ ID NO:5: Designed oligonucleotide primer designated as AS6 for amplifying human transferrin receptor cDNA.

SEQ ID NO:6: Designed oligonucleotide primer designated as AS4 for amplifying human transferrin receptor cDNA.

SEQ ID NO:7: Designed oligonucleotide primer designated as AS3 for amplifying human transferrin receptor cDNA.

SEQ ID NO:8: Designed oligonucleotide primer designated as AS2 for amplifying human transferrin receptor cDNA.

SEQ ID NO:9: Designed oligonucleotide primer designated as AS1 for amplifying human transferrin receptor cDNA.

SEQ ID NO:11: Designed oligonucleotide primer designated as AS5 for amplifying human transferrin receptor cDNA.

SEQUENCE LISTING

<110> Takara Shuzo Co., Ltd.

<120> DNA chips

<130> 38-43

<140> EP 00944440.7
<141> 2000-07-17

<150> JP 11-209782
<151> 1999-07-23

<160> 11

<210> 1
<211> 20
<212> DNA
<213> Artificial Sequence

<220>
<223> Designed oligonucleotide primer designated as CA13-S6 for
amplifying cDNA for human cancer-associated gene CA13.

<400> 1
gacagagcat cagaaccaga                                    20

<210> 2

<211> 18

<212> DNA

<213> Artificial Sequence

<220>

<223> Designed oligonucleotide primer designated as CA13-A4 for

amplifying cDNA for human cancer-associated gene CA13.

<400> 2

cttttcaatc catccacc                                                18

<210> 3

<211> 20

<212> DNA

<213> Artificial Sequence

<220>

<223> Designed oligonucleotide primer designated as S7 for amplifying

human transferrin receptor cDNA.

<400> 3

caagctagat cagcattctc                                              20

<210> 4

<211> 20

<212> DNA

<213> Artificial Sequence

<220>

<223> Designed oligonucleotide primer designated as AS7 for amplifying
human transferrin receptor cDNA.

<400> 4
tataccttta cctccaaaag                                        20

<210> 5
<211> 20
<212> DNA
<213> Artificial Sequence

<220>
<223> Designed oligonucleotide primer designated as AS6 for amplifying
human transferrin receptor cDNA.

<400> 5
acaaatctgt ctgttttctc                                        20

<210> 6
<211> 20
<212> DNA
<213> Artificial Sequence

<220>
<223> Designed oligonucleotide primer designated as AS4 for amplifying
human transferrin receptor cDNA.

<400> 6
ttgtctggac aggtatatta                                        20

<210> 7

<211> 20

<212> DNA

<213> Artificial Sequence

<220>

<223> Designed oligonucleotide primer designated as AS3 for amplifying

human transferrin receptor cDNA.

<400> 7

tcaacataca acgcaagatt                                    20

<210> 8

<211> 20

<212> DNA

<213> Artificial Sequence

<220>

<223> Designed oligonucleotide primer designated as AS2 for amplifying

human transferrin receptor cDNA.

<400> 8

gtcccatagc agatacttcc                                    20

<210> 9

<211> 20

<212> DNA

<213> Artificial Sequence

<220>

<223> Designed oligonucleotide primer designated as AS1 for amplifying

human transferrin receptor cDNA.

<400> 9

ccacatgact gttatcgcca                                              20

<210> 10

<211> 1048

<212> DNA

<213> Human

<400> 10

gacagagcat cagaaccaga ggcttggtcc cacaggccac ggaccaatgg cctgcagccg   60

tgacaacact cctgtcatat tggagtccaa aacttgaatt ctgggttgaa ttttttaaaa   120

atcaggtacc acttgatttc atatgggaaa ttgaagcagg aaatattgag ggcttcttga   180

tcacagaaaa ctcagaagag atagtaatgc tcaggacagg agcggcagcc ccagaacagg   240

ccactcattt agaattctag tgtttcaaaa cacttttgtg tgttgtatgg tcaataacat   300

ttttcattac tgatggtgtc attcacccat taggtaaaca ttccctttta aatgtttgtt   360

tgttttttga gacaggatct cactctgttg ccagggctgt agtgcagtgg tgtgatcata   420

gctcactgca acctccacct cccaggctca agcctcccga atagctggga ctacaggcgc   480

acaccaccat ccccggctaa tttttgtatt ttttgtagag acggggtttt gccatgttgc   540

caaggctggt ttcaaactcc tggactcaag aaatccaccc acctcagcct cccaaagtgc   600

taggattaca ggcatgagcc actgcgccca gcccttataa atttttgtat agacattcct   660

ttggttggaa gaatatttat aggcaataca gtcaaagttt caaaatagca tcacacaaaa   720

catgtttata aatgaacagg atgtaatgta catagatgac attaagaaaa tttgtatgaa   780

ataatttagt catcatgaaa tatttagttg tcatataaaa acccactgtt tgagaatgat   840

gctactctga tctaatgaat gtgaacgtgt agatgttttg tgtgtatttt tttaaatgaa   900

aactcaaaat aagacaagta atttgttgat aaatattttt aaagataact cagcatgttt 960

gtaaagcagg atacatttta ctaaaaggtt cattggttcc aatcacagct cataggtaga 1020

gcaaagaaag ggtggatgga ttgaaaag

<210> 11

<211> 20

<212> DNA

<213> Artificial Sequence

<220>

<223> Designed oligonucleotide primer designated as AS5 for amplifying

human transferrin receptor cDNA.

<400> 11

atacaacagt gggctggcag 20

## Claims

1. A DNA chip having DNAs being arrayed and immobilized in predetermined regions on a surface of a substrate, which is prepared by a method comprising immobilizing double-stranded DNAs onto an immobilization substrate under denaturing conditions.

2. The DNA chip according to claim 1, wherein the DNAs are immobilized onto the immobilization substrate using a DNA solvent containing a volatile acid or a salt thereof.

3. The DNA chip according to claim 2, wherein the volatile acid or a salt thereof is a volatile organic acid or a salt thereof.

4. The DNA chip according to claim 3, wherein the volatile organic acid or a salt thereof is acetic acid or a salt thereof.

5. The DNA chip according to any one of claims 1 to 4, wherein the immobilization substrate is a nonporous substrate.

6. The DNA chip according to claim 5, wherein the nonporous substrate is made of glass.

7. A DNA solvent for preparing a DNA chip containing a volatile acid or a salt thereof.

8. The DNA solvent according to claim 7, wherein the volatile acid or a salt thereof is a volatile organic acid or a salt thereof.

9. The DNA solvent according to claim 8, wherein the volatile organic acid or a salt thereof is acetic acid or a salt thereof.

10. The DNA solvent according to any one of claims 7 to 9, which further contains a surfactant.

11. A method for preparing a DNA chip having DNAs being arrayed and immobilized in predetermined regions on a surface of a substrate, the method comprising immobilizing double-stranded DNAs onto an immobilization substrate under denaturing conditions.

12. The method according to claim 11, wherein the DNAs are arrayed and immobilized in the predetermined regions on the immobilization substrate using a DNA solvent containing a volatile acid or a salt thereof.

13. The method according to claim 12, wherein the volatile acid or a salt thereof is a volatile organic acid or a salt thereof.

14. The method according to claim 13, wherein the volatile organic acid or a salt thereof is acetic acid or a salt thereof.

15. The method according to any one of claims 11 to 14, wherein the immobilization substrate is a nonporous substrate.

16. The method according to claim 15, wherein the nonporous substrate is made of glass.
